# EUROPEAN PATENT SPECIFICATION

(11) **EP 4 135 773 B1**
(45) Date of publication and mention of the grant of the patent: **19.11.2025**
(21) Application number: 21724037.3
(22) Date of filing: 15.04.2021
(51) Int. Cl.: A61K 47/61, C08B 37/08, A61P 35/00, C08L 5/08

(54) **PROCESS FOR THE SYNTHESIS OF A CONJUGATE OF HYALURONIC ACID AND PACLITAXEL**
VERFAHREN ZUR HERSTELLUNG EINES KONJUGATS HYALURONICSÄURE UND PACLITAXEL
PROCÉDÉ POUR LA SYNTHÈSE D'UN CONJUGUÉ DE L'ACIDE HYALURONIQUE ET DE PACLITAXEL

(30) Priority: 17.04.2020 IT 202000008209
(43) Date of publication of application: 22.02.2023
(60) Divisional application: 25207960.3
(73) Proprietor: Fidia Farmaceutici S.p.A., 35031 Abano Terme (PD) (IT)
(72) Inventor: CAMPISI, Monica, 35031 Abano Terme (PD) (IT); GUARISE, Cristian, 35031 Abano Terme (PD) (IT); PIZZOCARO, Carlo, 35031 Abano Terme (PD) (IT)
(74) Representative: Bird & Bird Società tra Avvocati S.r.l.
(86) International application number: PCT/IB2021/053096
(87) International publication number: WO 2021/209935

(56) References cited:
- WO-A1-2009/003624
- WO-A1-2009/130564
- WO-A1-2020/084525
- WO-A2-2004/035629
- FRANCESCA LEONELLI ET AL: "Design, Synthesis and Applications of Hyaluronic Acid-Paclitaxel Bioconjugates", MOLECULES, vol. 13, no. 2, 12 February 2008 (2008-02-12), pages 360 - 378, XP055757515, ISSN: 1433-1373, DOI: 10.3390/molecules13020360
- FRANCESCA LEONELLI ET AL: "A New and Simply Available Class of Hydrosoluble Bioconjugates by Coupling Paclitaxel to Hyaluronic Acid through a 4-Hydroxybutanoic Acid Derived Linker", HELVETICA CHIMICA ACTA, vol. 88, no. 1, 2005, pages 154 - 159, XP055270146, ISSN: 0018-019X, DOI: 10.1002/hlca.200490289
- SILVIA ARPICCO ET AL: "Hyaluronic Acid Conjugates as Vectors for the Active Targeting of Drugs, Genes and Nanocomposites in Cancer Treatment", MOLECULES, vol. 19, no. 3, 17 March 2014 (2014-03-17), pages 3193 - 3230, XP055414782, DOI: 10.3390/molecules19033193
- ZHANG HAIQUN ET AL: "Current research on hyaluronic acid-drug bioconjugates", EUROPEAN JOURNAL OF MEDICINAL CHEMISTRY, vol. 86, 27 August 2014 (2014-08-27), pages 310 - 317, XP029048584, ISSN: 0223-5234, DOI: 10.1016/J.EJMECH.2014.08.067
- ROSATO A ET AL: "HYTAD1-p20: A new paclitaxel-hyaluronic acid hydrosoluble bioconjugate for treatment of superficial bladder cancer", UROLOGIC ONCOLOGY: SEMINARS AND ORIGINAL INVESTIGATIONS, vol. 24, no. 3, May 2006 (2006-05-01), pages 207 - 215, XP028071920, ISSN: 1078-1439, [retrieved on 20060501], DOI: 10.1016/J.UROLONC.2005.08.020
- SARAVANAKUMAR G ET AL: "Hyaluronic acid-based conjugates for tumor-targeted drug delivery and imaging", vol. 10, no. 1, 31 December 2013 (2013-12-31), pages 17 - 31, XP009514109, ISSN: 1550-7033, Retrieved from the Internet <URL:1077952576> DOI: 10.1166/JBN.2014.1761
- ILARIA DE STEFANO ET AL: "Hyaluronic acid-paclitaxel: effects of intraperitoneal administration against CD44(+) human ovarian cancer xenografts", CANCER CHEMOTHERAPY AND PHARMACOLOGY, vol. 68, no. 1, 17 September 2010 (2010-09-17), pages 107 - 116, XP019919939, ISSN: 1432-0843, DOI: 10.1007/S00280-010-1462-2

## Description

### OBJECT OF THE INVENTION

The present invention describes the industrial synthesis process of a hyaluronic acid-paclitaxel conjugate obtained according to a synthesis process between hyaluronic acid (HA) molecules and paclitaxel by introducing the 4-bromobutyric acid spacer/linker between the hyaluronic acid and the above-mentioned chemotherapeutic agent, according to an original synthesis and purification process which ensures the purity of the conjugate in its final form.

### FIELD OF THE INVENTION

The paclitaxel (Taxol^{®}) is an anti-cancer agent performing its antiproliferative action by acting on the organization of microtubules of the cellular cytoskeletal system: by inhibiting the depolarisation of the above-mentioned microtubules, it prevents the normal dynamic reorganization thereof, which occurs during the mitotic cell division (Manfredi JJ et al., J Cell Biol, 1982, 94:688-696).

The main therapeutic indications of paclitaxel are its use in the therapy of advanced breast cancer, in the therapy of lung cancer, in the treatment of ovarian cancer, of bladder, prostate and endometrium cancer.

The paclitaxel is a water-insoluble compound and, consequently, the pharmaceutical composition in which it is currently deployed comprises mixing it with Cremophor EL - ethyl alcohol. (Pfeifer RW et al., Am J Hasp Pharm, 1993, 50:2520-2521) in a 1:1 ratio, and it is in this formulation that it is normally administered by continuous intravenous infusion. The presence of Cremophor EL represents the main cause of adverse reactions which normally occur during the administration of paclitaxel, i.e. simple attacks of urticaria, dyspnea and bronchospasm, up to anaphylactic shock. For this reason, all patients treated with the paclitaxel-Cremophor EL pharmaceutical composition must follow, before starting therapy, a pre-medication protocol which consists of the administration of dexamethasone, possibly associated with an antihistamine.

We can therefore state that the formulation of Taxol^{®} which is currently in clinical use (and its administration route) constitutes a limit to the therapeutic efficacy thereof. This is the reason why research is currently oriented both towards the synthesis of prodrugs/water-soluble paclitaxel conjugates and towards new formulations of the above-mentioned anti-cancer drug.

Paclitaxel is an alkaloid currently obtained semi-synthetically from one of its precursors derived from *Taxus Baccata* needles. Chemically, it consists of a 15-carbon-atom taxane ring bound to an oxyethane ring in C4 and C5 position, whereas in C13 position an ester bond is present, which is considered indispensable for its anti-cancer activity.

To overcome the above-mentioned problems consequent to the type of formulation, attempts have been made, for example, to encapsulate said anti-cancer agent in liposomes, nanocapsules and microspheres constituted by a polymer wall formed by biodegradable co-polymers such as polylactic acid, or non-biodegradable co-polymers such as ethylene-vinyl-acetate, or microspheres formed by polyphosphoester loaded with paclitaxel to create a system for the prolonged release of drug at the treatment site (Nuijen B et al., Investigational New Drugs, 2001, 19:143-153).

However, these new systems for the encapsulation of paclitaxel have presented problems of stability, production and reproducibility.

Moreover, various attempts have been made to solubilize the above-mentioned drug with cyclodextrins, but the new formulations did not give the desired results (Nuijen B et al., Investigational New Drugs, 2001, 19:143-153).

Chemical research into new paclitaxel formulations which would render the drug more water-soluble while maintaining its efficacy as anti-cancer agent unaltered, has led to the synthesis of new analogues modified at the C2' and C7 positions and, furthermore, to the preparation of new conjugates also defined as prodrugs.

With this in mind, several attempts have been made to synthesize new prodrugs which have led, for example, to the preparation of drugs such as acetyl-paclitaxel (Mellado W et al., Biochem Biophys Res Commun, 1984, 124(2): 329-336) or to the synthesis of new esters of the above-mentioned drug with succinate, glutarate and sulphonic acid on the carbon at C2' position, esters which proved to be, however, unstable in aqueous environment.

Furthermore, also the use of PEG (polyethyleneglycol) for the derivatization of the above-mentioned chemotherapeutic agent by esterification of paclitaxel at C2' position is known. Such new molecule proved to be highly water-soluble but also with a limited stability.

Lastly, a new *"delivery system"* of the above-mentioned drug was also developed, which is obtained by conjugating paclitaxel with serum albumin protein (HSA): the paclitaxel-HSA conjugate proved to be highly water-soluble, but without reliable efficacy data with respect to paclitaxel *per se* (Nuijen B et al., Investigational New Drugs, 2001, 19:143-153).

Recently a new release system of paclitaxel esterified with previously modified hyaluronic acid (HA), i.e. derivatized with hydrazide molecules bound to the carboxyl group of the HA by an amide bond, has been synthesized (Luo Y et al., Biomacromolecules, 2000, 1(2):208-218) (US 5,874,417). A new paclitaxel delivery system was thus obtained, which enables the drug to directly reach the membrane surface of the target tumor cell, which is characterized by an overexpression of HA receptor, CD44.

In the attempt to develop all the potentialities of the association of paclitaxel with HA, a new conjugate between HA and the chemotherapeutic agent was eventually studied and synthesized, in which HA and the chemotherapeutic agent are covalently bound to each other indirectly by a spacer/linker, such as for example the bromobutyric acid, which renders the final product more water-soluble and therefore easy to administer (EP2045270).

The scope of the present invention is to identify a new process for the preparation and purification which is particularly efficient and overcomes the drawbacks of the prior art highlighted above.

### DETAILED DESCRIPTION OF THE INVENTION

Object of the present invention is therefore an original process for the preparation and purification of the hyaluronic acid-paclitaxel (HA-paclitaxel) conjugate which comprises or consists of the following steps:
a) activation in a solution of paclitaxel in an organic solvent, preferably dichloromethane, containing a 4-bromobutyric acid spacer/linker, of the carboxyl group of the above-mentioned 4-bromobutyric acid spacer/linker by means of an activating agent, preferably a carbodiimide, more preferably 1-ethyl-3-(3-dimethylaminopropyl)carbodiimide hydrochloride (EDC, CAS number 25952-53-8), in the presence of a catalyst, preferably DMAP (4-dimethylaminopyridine) obtaining the intermediate of the HA-paclitaxel conjugate (Br-C₄-paclitaxel);
b) crystallization of the intermediate obtained from the previous step a), in an organic solvent, preferably N-heptane, subsequently its filtration and drying;
c) addition of the intermediate obtained in the previous step b) to the TBA (tetrabutylammonium) salt of HA in DMSO;
d) purification of the HA-paclitaxel conjugate thus obtained which has an ester bond between the carboxyl of the HA polysaccharide and the spacer/linker, said spacer/linker being in turn bound by an ester bond through its carboxyl to the hydroxyl group at carbon C2' of paclitaxel, by precipitation with an ethanol/water solution containing the NaBr salt, at least one subsequent washing in ethanol/water and a further washing in ethanol, pre-drying at 40°C and passage in a saturated chamber of water vapour at 40°C and at atmospheric pressure, finally drying at 40°C under vacuum.

It is also disclosed (not object of the invention) the HA-paclitaxel conjugate having a derivatization or esterification degree within the range of 15% to 21% w/w, preferably within the range of 16% to 20% w/w, wherein the starting hyaluronic acid is of a fermentative origin with an average weight molecular weight ranging from 140,000 to 250,000 Da, said conjugate being in the form of a fine and homogeneous powder, with a degree of purity greater than 98% by weight on the dry product and containing a quantity of di-Br-paclitaxel of less than 1% by weight. It is disclosed (not object of the invention) also the HA-paclitaxel conjugate in the form of a fine and homogeneous powder containing less than 1% by weight of di-Br-paclitaxel, prepared and purified as described and therefore extremely pure with respect to the same conjugate obtained with the process according to EP2045270. It is disclosed (not object of the present invention) also a pharmaceutical composition essentially consisting of the conjugate prepared and purified with the process described above, associated with pharmacologically acceptable diluents/excipients, preferably a pharmaceutical composition formulated in sterile, isotonic water, containing 5% w/v of glucose, for use in the treatment of advanced breast cancer, lung cancer, ovarian cancer, bladder cancer, prostate and endometrial cancer and mesothelioma.

The composition is used in the treatment of all cancer forms in which paclitaxel, all known above-mentioned derivatives of such drug and taxanes in general are normally deployed.

The present invention therefore describes and claims the preparation and purification of the HA-paclitaxel conjugate according to an original synthesis process consisting of the introduction of a spacer/linker between the hyaluronic acid and the chemotherapeutic agent above, through the formation of the ester bond between the carboxyl of the above-mentioned polysaccharide and the spacer/linker, said spacer/linker being in turn bound (always by an ester bond) through its carboxyl to the hydroxyl group at carbon C2' of paclitaxel, wherein the spacer/linker used for the synthesis of the above-mentioned conjugate is the 4-bromobutyric acid.

Such new synthesis process of the HA-paclitaxel conjugate differs from EP2045270 as it allows:
- the formation of the conjugate with a greater degree of purity;
- the elimination of the final dialysis;
- the reduction of solvents use;
- the obtainment of the raw material (HA-paclitaxel conjugate) as a fine and homogeneous powder, easily sterilizable by filtration once formulated in an aqueous solvent;
- the reduction of synthesis times.

The HA-paclitaxel conjugate thus prepared and purified has a derivatization degree within the range of 15% to 21% weight/weight (w/w), preferably within the range of 16% to 20% w/w.

The degree of derivatization or esterification of the above-mentioned conjugate is defined below as the percentage by weight of paclitaxel vs. the weight of HA-paclitaxel, therefore 100mg of conjugate with derivatization degree comprised between 15% and 21% w/w, will contain 15mg, 16mg, 17mg, 18mg, 19mg, or 20mg, or 21mg of chemotherapeutic paclitaxel depending on the indicated derivatization degree (to further exemplify this, the derivatization degree at 20% w/w contains 20mg of paclitaxel per 100mg of conjugate); it is however obvious to the skilled person that, at the end of such industrial synthesis processes, a small variation in the weight ratios between the molecules can occur, therefore hereinafter the Applicant, describing the derivatization range of the above-mentioned conjugate comprised from 15% to 21% w/w, intends to disclose all the reported percentage values comprising ±1%: by way of example, the degree of 20% w/w is accordingly intended to be as 20% ± 1%.

The HA used for the synthesis of such HA-paclitaxel conjugate can derive from any source, for example by extraction from rooster combs (e.g. according to EP0138572 or WO2018020458), or by biotechnological route (e.g. according to EP2614088 or EP2614087), or preferably by fermentative route (e.g. according to EP0716688), and have an average weight molecular weight (MW) ranging from 400 to 3x10⁶Da, particularly from 400 to 1x 10⁶Da, even more particularly from 140,000 to 250,000 Da (average weight molecular weight means the one calculated with the *"intrinsic viscosity"* method (Terbojevich et al., Carbohydr Res, 1986, 363-377). The HA-paclitaxel described and claimed by the Applicant has a derivatization degree within the range of 15% to 21% w/w, preferably within the range of 16% to 20% w/w, preferably prepared from an HA of fermentative origin with an average weight molecular weight ranging from 140,000 to 250,000 Da.

The HA-paclitaxel conjugate described above is prepared and purified according to the above-mentioned original synthesis process modified with respect to the prior art and thus detailed below:
- in a solution of paclitaxel in an organic solvent, preferably dichloromethane, containing the 4-bromobutyric acid spacer/linker, the carboxyl group of the 4-bromobutyric acid is activated by an activating agent, such as a carbodiimide, preferably 1-ethyl-3-(3-dimethylaminopropyl)carbodiimide hydrochloride (EDC), in the presence of a catalyst, preferably DMAP (4-dimethylaminopyridine), the formation of an ester between the hydroxyl function at the C2' carbon of paclitaxel and the carboxyl of the spacer/linker is thus obtained for the formation of the intermediate of the HA-paclitaxel conjugate, wherein the bond between said spacer/linker and the paclitaxel is an ester bond;
- the obtained intermediate is crystallized by adding an organic solvent, preferably N-heptane, to the solution above, then isolated by filtration and dried; the dried intermediate is thus obtained, which is then analyzed in order to evaluate the degree of purity through the determination in HPLC of the di-Br-paclitaxel which, thanks to the above-mentioned crystallization step, is drastically reduced, proving to be less than 1% by weight, consequently the purity of the product of such synthesis proves to be greater than 98% w/w;
- subsequently, by direct contact of said intermediate with the TBA (tetrabutylammonium) salt of HA in dimethyl sulfoxide (DMSO) (the DMSO allows the dissolution of such intermediate and optimizes the reaction of said synthesis compared to N-methyl-2-pyrrolidone (NMP) and to dimethylformamide (DMF)), the nucleophilic substitution of the COO- of HA to the carbon bound to the bromine of the spacer/linker is obtained. In this way, the ester bond between the HA and the spacer/ linker previously bound to paclitaxel is formed;
- the obtained conjugate is thus purified by precipitation with an ethanol/water solution containing NaBr (T 40°C), then washed once or more in ethanol/water and subsequently washed in ethanol, then pre-dried at 40°C, left at 40°C in a saturated chamber of water vapour at atmospheric pressure, finally dried at 40°C under vacuum.

The Applicant surprisingly found that NaBr facilitates the precipitation of the conjugate and therefore its purification, at the same time the NaBr is in turn easily removed by the subsequent washings of the conjugate with the hydroalcoholic solutions above.

The pre-drying steps and passage in a saturated chamber of water vapour at atmospheric pressure are carried out at 40°C, for a time of at least 8 hours, whereas the drying step is carried out at 40°C, under vacuum, for a time of at least 16 hours. This purification step of the conjugate excludes the dialysis process.

The obtained final product is in the form of a fine and homogeneous powder, it is analyzed via HPLC to evaluate its degree of derivatization or esterification of the carboxyl, and its filterability.

The HA-paclitaxel conjugate prepared and purified as reported above thus proves to be free of contaminating agents mainly represented by:
- di-Br-paclitaxel: a fully unwanted reaction byproduct due to the esterification reaction of two 4-bromobutyric acid molecules with a paclitaxel molecule on two different hydroxyls. The reduction of the content of this secondary product is a remarkable improvement of the synthesis and purification process of the conjugate, as the di-Br-paclitaxel reacts with two carboxyl functions of one or two HATBA molecules and determines therefore the formation of a cross-linked completely lacking therapeutic efficacy which compromises sterilization by filtration with 0.2µ filters of the pharmaceutical composition containing the conjugate (not object of the invention);
- residues of NaBr, of solvents and of activating agents which are eliminated thanks to the step of crystallization of the intermediate in N-heptane, thanks to the step of precipitation in ethanol and the subsequent washings in hydroalcoholic solution of the HA-paclitaxel final product. The process of crystallization in N-heptane may in fact occur and therefore involve only the intermediate, leading in this way to a pure product which is easy to isolate from residues of various kinds present in the reaction medium.

Thanks to all the above-listed steps, an extremely pure fine powder conjugate is obtained, without the need to carry out a final dialysis process, therefore determining also a considerable saving of solvents.

The synthesis and purification process of the HA-paclitaxel conjugate object of the invention therefore differs from EP2045270 as it allows:
- the formation of the conjugate with a greater degree of purity;
- the elimination of the final dialysis;
- the reduction of solvents use;
- the obtainment of the raw material as a fine and homogeneous powder, easily sterilizable by filtration once formulated in an aqueous solvent;
- the overall reduction of its synthesis times.

In order to better illustrate the scopes and advantages of the present invention an example of preparation and purification of the HA-paclitaxel conjugate is provided, which, however, does not constitute in any way a limitation of the scope of the claims.

### Example 1: preparation and purification of the HA-paclitaxel conjugate with a derivatization degree of 18% w/w

Preparation of the ester derivative of HA (HA used for such synthesis has an average weight molecular weight ranging from 160,000 to 230,000 Da) with the paclitaxel having a degree of derivatization, i.e. of the esterification of the carboxyl, of 18% w/w.

110.1 mg of 4-bromobutyric acid, 151.9 mg of EDC and 19.5 mg of DMAP were added to 308.7 mg of paclitaxel dissolved in 15 ml of dichloromethane, under stirring at 10°C for at least 40 minutes. Subsequently, water was added to the solution thus obtained; after stirring for some minutes, the organic phase is allowed to separate. The organic phase was collected, whereas the aqueous phase (containing the bromide residue and salts) was eliminated. 25 ml of N-epthane were added to the organic phase thus obtained, at 10°C, obtaining the crystallization of the intermediate product, then isolated by filtration and hence dried. In this way, 301 mg of dried intermediate product have been obtained.

300 mg of such intermediate have been added to a solution of 1000 mg of HA-TBA (salt of HA with tetrabutylammonium) dissolved in 44 ml of DMSO. After 1 day of reaction at T 40°C, 50 ml of DMSO have been added to the solution. After 1 hour, a solution of ethanol/water in a 96/4 ratio containing NaBr at 2% w/w has been slowly added a drop at a time, thus obtaining the precipitation of the produced conjugate that was washed many times in ethanol/water (8.5/1.5), finally re-washed with 100% ethanol and then pre-dried at 40°C for 8 hours. The pre-dried product has been subsequently left at 40°C in a saturated chamber of water vapour at atmospheric pressure for further 8 hours, and finally dried at 40°C under vacuum for at least 16 hours and however until complete drying.

The obtained HA-paclitaxel conjugate has been then analysed through HPLC analysis (Rosato A., Urologic Oncology: Seminars and Original Investigations, 2006, 24: 207-215) to check the effective derivatization degree resulted to be 18% w/w; the HPLC analysis on the dried intermediate (Theodoridis G. et al., Application Note, August 1999:40-44) determined the quantity of di-Br-paclitaxel which resulted to be less than 1% by weight (as demonstrated below), the purity degree of the conjugate resulted to be greater than 98% by weight on the dry product.

The HA-paclitaxel conjugate obtained as describe above comes as white-yellowish powder, homogeneous and hygroscopic, easily sterilizable by filtration with 0.2µ filters, when reconstituted as pharmaceutical composition by aqueous medium.

### Example 2 comparative: preparation and purification of the HA-paclitaxel conjugate according to WO2004035629 (EP2045270)

### Preparation of the ester derivative of HA with paclitaxel with a degree of the esterification of the carboxyl of 18% w/w.

In order to demonstrate that the process object of the invention overcomes the prior art represented by the process described in WO2004/035629, the Applicant prepared the HA-paclitaxel conjugate according to the Example 6 of WO2004/035629, modifying the weight concentration of the different components, in order to prepare an HA-paclitaxel conjugate with a degree of esterification of the carboxyl of 18% w/w, to compare such derivative in terms of purity and filterability with the HA-paclitaxel conjugate of the Example 1.

The HA used has an average weight molecular weight ranging between 160,000 and 230,000 Da as per Example 1.

Shortly, 117.2 mg of 4-bromobutyric acid and 614.1 mg of EDC were added to 308.7 mg of paclitaxel dissolved in 15 ml of dichloromethane. Subsequently, water was added to the solution to eliminate all the bromide and carbodiimide. Sodium sulphate was added to the organic solution thus obtained to dehydrate, while the solvent was eliminated through a rotavapor. Finally, 363 mg of dried intermediate product were obtained.

315 mg of intermediate thus obtained were added to 1 g of HA-TBA dissolved in anhydrous NMP, the solution was stirred at room temperature for 7 days, after which 20 ml of double-distilled water and 4 ml of a saturated NaCl solution were added. It was stirred for 1 hour to enable the exchange of sodium with the TBA ion. Subsequently, ethanol was added a drop at a time and the filamentous product thus obtained was dissolved in water, dialysed, and lastly lyophilized.

The HA-paclitaxel conjugate obtained according to Example 2 was then analysed through HPLC to check the effective derivatization degree resulted to be 18% w/w. The two conjugates from Example 1 and Example 2 were therefore compared to verify their purity in terms of quantity of di-Br-paclitaxel (cross-linked reaction byproduct) present and determined in the dried intermediate from Example 1 and 2 (HPLC analysis on the dried intermediate, Theodoridis G. et al., Application Note, August 1999:40-44), and the filterability through cellulose acetate filters (Minisart^{®}, 0,2µ) which ensure to the skilled person the sterility of the product, measured at a pressure of 2 atm.

### Filterability Test:

For the test execution a small steel tank (Sartorius Stedim Vol. 0.22 L) with a compressed air inlet hole (regulated by a manometer) on the upper part, and an outlet hole on the lower part was used. The tank was loaded with 15 ml of a solution of HA-paclitaxel dissolved to 12 mg/ml in glucose at 5% w/w. Under the tank, on the outlet hole, a 0.2µ filter (Minisart, CE, Sterile) was mounted. The flow of compressed air was regulated in order to obtain a pressure of about 1.5 Bar inside the steel tank, with the purpose of pushing the solution to flow through the sterile filter. A chronometer was started at the time of beginning of the filtration and, placing a graduated cylinder under the outlet hole of the filter, the volume filtered over time was measured.

### Results:

### HA-paclitaxel conjugate prepared according to Example 1:

di-Br-paclitaxel w/w (percentage by weight vs intermediate weight): **0.8%**
Filterability (of a solution of 12mg/ml conjugate in a glucose solution 5% w/w): **4.5 ml/minute**

### HA-paclitaxel conjugate prepared according to Example 2:

di-Br-paclitaxel w/w: **5.2%**
Filterability: **0.04 ml/minute.**

In conclusion, the obtained results show that the HA-paclitaxel conjugated produced according to the process object of the invention is characterized by:
- a very high purity degree, being the quantity of di-Br-paclitaxel entirely negligible.;
- a high filtration capacity through 0.2µ filters which, as known to the skilled person, ensure the sterility of the filtrate (a filtration capacity of 0.04 ml/minute is an indication of a little/badly filterable product, therefore NOT sterilizable through 0.2µ filters, making necessary the choice of an alternative sterilization method, such as heat or radiation sterilization, both methods however determining, as widely known, the partial degradation of the products subjected to such processes, especially of ester derivatives).

Finally, the process object of the invention results in a significant reduction of the quantity of the solvent used since in such process the purification dialysis of the conjugate is not carried out, planned instead in the process known to the skilled person, with a substantial reduction of the preparation time of the conjugate as per example 1-2 in comparison.

The obtained conjugate therefore resulted to have optimal characteristics compared to the one obtained as for the prior art (WO2004035629) with, in addition, considerable cost and time savings.

## Claims

1. A process for the preparation and purification of a hyaluronic acid-paclitaxel (HA-paclitaxel) conjugate which comprises or consists of the following steps:
a) activation in a solution of paclitaxel in an organic solvent, preferably dichloromethane, containing a 4-bromobutyric acid spacer/linker, of the carboxyl group of the above-mentioned 4-bromobutyric acid spacer/linker by means of an activating agent in the presence of a catalyst, preferably DMAP (4-dimethylaminopyridine) obtaining the intermediate of the HA-paclitaxel conjugate;
b) crystallization of the intermediate obtained from the previous step a), in an organic solvent, preferably N-heptane, subsequently its filtration and drying;
c) addition of the intermediate obtained in the previous step b) to the TBA (tetrabutylammonium) salt of HA in DMSO;
d) purification of the HA-paclitaxel conjugate thus obtained which has an ester bond between the carboxyl of the HA polysaccharide and the spacer/linker, said spacer/linker being in turn bound by an ester bond through its carboxyl to the hydroxyl group at carbon C2' of paclitaxel, by precipitation with an ethanol/water solution containing the NaBr salt, at least one subsequent washing in ethanol/water and a further washing in ethanol, pre-drying at 40°C and passage in a saturated chamber of water vapour at 40°C and at atmospheric pressure, finally drying at 40°C under vacuum.

2. The process according to claim 1, wherein in the activation step a), the activating agent is a carbodiimide, preferably 1-ethyl-3-(3-dimethylaminopropyl) carbodiimide hydrochloride (EDC).

3. The process according to one or more of the previous claims, wherein in the activation step a), the catalyst is 4-dimethylaminopyridine (DMAP).

4. The process according to one or more of the previous claims, wherein in the crystallization step b), the solvent is N-heptane.

5. The process according to one or more of the previous claims, wherein in the purification step d), the pre-drying and the passage in a saturated chamber of water vapour at atmospheric pressure are carried out at 40°C, for a time of at least 8 hours, whereas the drying step is carried out at 40°C, under vacuum, for a time of at least 16 hours.

6. The process according to one or more of the previous claims, wherein the starting hyaluronic acid has an average weight molecular weight ranging from 400 to 3x10⁶Da, in particular from 400 to 1x10⁶Da, even more particularly from 140,000 to 250,000 Da.

7. The process according to one or more of the previous claims, wherein the starting hyaluronic acid is of an extractive origin from rooster combs, biotechnological or fermentative origin, preferably fermentative.

8. The process according to one or more of the previous claims, wherein the starting hyaluronic acid is of a fermentative origin with an average weight molecular weight ranging from 140,000 to 250,000 Da.

## Patentansprüche

1. Verfahren zur Herstellung und Reinigung eines Hyaluronsäure-Paclitaxel (HA-Paclitaxel)-Konjugats, das die folgenden Schritte umfasst oder aus diesen besteht:
a) Aktivierung der Carboxylgruppe des oben erwähnten 4-Brombuttersäure-Spacers/Linkers in einer Lösung von Paclitaxel in einem organischen Lösungsmittel, vorzugsweise Dichlormethan, das einen 4-Brombuttersäure-Spacer/Linker enthält, mit Hilfe eines Aktivierungsmittels in Gegenwart eines Katalysators, vorzugsweise DMAP (4-Dimethylaminopyridin), wodurch das Zwischenprodukt des HA-Paclitaxel-Konjugats erhalten wird;
b) Kristallisation des aus dem vorhergehenden Schritt a) erhaltenen Zwischenprodukts in einem organischen Lösungsmittel, vorzugsweise N-Heptan, anschließend dessen Filtration und Trocknung;
c) Zugabe des im vorhergehenden Schritt b) erhaltenen Zwischenprodukts zum TBA (Tetrabutylammonium)-Salz von HA in DMSO;
d) Reinigung des so erhaltenen HA-Paclitaxel-Konjugats, das eine Esterbindung zwischen dem Carboxyl des HA-Polysaccharids und dem Spacer/Linker aufweist, wobei der Spacer/Linker wiederum durch eine Esterbindung über sein Carboxyl an die Hydroxylgruppe an Kohlenstoff C2' von Paclitaxel gebunden ist, durch Ausfällen mit einer Ethanol/WasserLösung, die das NaBr-Salz enthält, mindestens ein anschließendes Waschen in Ethanol/Wasser und ein weiteres Waschen in Ethanol, Vortrocknen bei 40 °C und Durchgang in einer gesättigten Wasserdampfkammer bei 40 °C und bei Atmosphärendruck, schließlich Trocknung bei 40 °C unter Vakuum.

2. Verfahren nach Anspruch 1, wobei im Aktivierungsschritt a) das Aktivierungsmittel ein Carbodiimid, vorzugsweise 1-Ethyl-3-(3-dimethylaminopropyl) carbodiimidhydrochlorid (EDC) ist.

3. Verfahren nach einem oder mehreren der vorhergehenden Ansprüche, wobei im Aktivierungsschritt a) der Katalysator 4-Dimethylaminopyridin (DMAP) ist.

4. Verfahren nach einem oder mehreren der vorhergehenden Ansprüche, wobei im Kristallisationsschritt b) das Lösungsmittel N-Heptan ist.

5. Verfahren nach einem oder mehreren der vorhergehenden Ansprüche, wobei im Reinigungsschritt d) die Vortrocknung und der Durchgang in einer gesättigten Wasserdampfkammer bei Atmosphärendruck bei 40 °C für eine Zeit von mindestens 8 Stunden durchgeführt werden, während der Trocknungsschritt bei 40 °C unter Vakuum für eine Zeit von mindestens 16 Stunden durchgeführt wird.

6. Verfahren nach einem oder mehreren der vorhergehenden Ansprüche, wobei die Ausgangshyaluronsäure ein mittleres Molekulargewicht im Bereich von 400 bis 3x10⁶Da, insbesondere von 400 bis 1x10⁶Da, noch weiter besonders von 140.000 bis 250.000 Da aufweist.

7. Verfahren nach einem oder mehreren der vorhergehenden Ansprüche, wobei die Ausgangshyaluronsäure extraktiven Ursprungs aus Hahnkämmen, biotechnologischen oder fermentativen Ursprungs, vorzugsweise fermentativen Ursprungs, ist.

8. Verfahren nach einem oder mehreren der vorhergehenden Ansprüche, wobei die Ausgangshyaluronsäure fermentativen Ursprungs mit einem durchschnittlichen Molekulargewicht im Bereich von 140.000 bis 250.000 Da ist.

## Revendications

1. Procédé de préparation et de purification d'un conjugué acide hyaluronique-paclitaxel (HA-paclitaxel) qui comprend ou consiste en les étapes suivantes :
a) activation dans une solution de paclitaxel dans un solvant organique, de préférence le dichlorométhane, contenant un espaceur/lieur d'acide 4-bromobutyrique, du groupe carboxyle de l'espaceur/lieur d'acide 4-bromobutyrique susmentionné au moyen d'un agent activateur en présence d'un catalyseur, de préférence la DMAP (4-diméthylaminopyridine), obtenant l'intermédiaire du conjugué HA-paclitaxel ;
b) cristallisation de l'intermédiaire obtenu à l'étape a) précédente, dans un solvant organique, de préférence le N-heptane, puis filtration et séchage ;
c) addition de l'intermédiaire obtenu à l'étape b) précédente au sel TBA (tétrabutylammonium) de HA dans du DMSO ;
d) purification du conjugué HA-paclitaxel ainsi obtenu, qui présente une liaison ester entre le carboxyle du polysaccharide HA et l'espaceur/lieur, ledit espaceur/lieur étant à son tour lié par une liaison ester, à travers son carboxyle, au groupe hydroxyle du carbone C2' du paclitaxel, par précipitation avec une solution éthanol/eau contenant le sel NaBr, au moins un lavage ultérieur dans l'éthanol/eau et un autre lavage dans l'éthanol, pré-séchage à 40°C et passage dans une chambre saturée de vapeur d'eau à 40°C et à la pression atmosphérique, enfin séchage à 40°C sous vide.

2. Procédé selon la revendication 1, dans lequel, à l'étape d'activation a), l'agent d'activation est un carbodiimide, de préférence le chlorhydrate de 1-éthyl-3-(3-diméthylaminopropyl) carbodiimide (EDC).

3. Procédé selon l'une ou plusieurs des revendications précédentes, dans lequel, à l'étape d'activation a), le catalyseur est la 4-diméthylaminopyridine (DMAP).

4. Procédé selon l'une ou plusieurs des revendications précédentes, dans lequel, à l'étape de cristallisation b), le solvant est le N-heptane.

5. Procédé selon l'une ou plusieurs des revendications précédentes, dans lequel, dans l'étape de purification d), le pré-séchage et le passage dans une chambre saturée de vapeur d'eau à la pression atmosphérique sont effectués à 40°C, pendant une durée d'au moins 8 heures, tandis que l'étape de séchage est effectuée à 40°C, sous vide, pendant une durée d'au moins 16 heures.

6. Procédé selon l'une ou plusieurs des revendications précédentes, dans lequel l'acide hyaluronique de départ a un poids moléculaire moyen allant de 400 à 3x10⁶Da, en particulier de 400 to 1x10⁶Da, encore plus particulièrement de 140 000 à 250 000 Da.

7. Procédé selon l'une ou plusieurs des revendications précédentes, dans lequel l'acide hyaluronique de départ est d'origine extractive à partir de crêtes de coq, d'origine biotechnologique ou fermentaire, de préférence fermentaire.

8. Procédé selon l'une ou plusieurs des revendications précédentes, dans lequel l'acide hyaluronique de départ est d'origine fermentaire avec un poids moléculaire moyen compris entre 140 000 et 250 000 Da.
